# EUROPEAN PATENT SPECIFICATION

(11) **EP 0 813 879 B1**
(45) Date of publication and mention of the grant of the patent: **30.10.2002**
(21) Application number: 97110139.9
(22) Date of filing: 20.06.1997
(51) Int. Cl.: A61K 47/18, A61K 47/22, A61K 9/00

(54) **Drug administration composition for iontophoresis**
Mittel zur Arzneistoffverabreichung durch Iontophorese
Composition pour l'administration d'un médicament par iontophorèse

(30) Priority: 20.06.1996 JP 18148096
(43) Date of publication of application: 29.12.1997
(73) Proprietor: HISAMITSU PHARMACEUTICAL CO. INC., Tosu-shi, Saga-ken 841-0017 (JP)
(72) Inventor: Higo, Naruhito, c/o Tsukuba Laboratory, Tsukuba-shi, Ibaraki-ken 305 (JP); Adachi, Hirotoshi, c/o Tsukuba Laboratory, Tsukuba-shi, Ibaraki-ken 305 (JP); Mori, Kenji, c/o Tsukuba Laboratory, Tsukuba-shi, Ibaraki-ken 305 (JP); Iga, Katsumi, Suita-shi, Osaka-fu 565 (JP)
(74) Representative: Goddar, Heinz J., Dr.

(56) References cited:
- EP-A- 0 747 092
- WO-A-93/10163
- US-A- 4 593 053
- US-A- 5 135 480

## Description

### BACK GROUND OF THE INVENTION

### Field of The Invention

This invention relates to a drug administration composition which is adapted for use in iontophoresis and which is useful for transdermal or transmucosal delivery of drugs or biologically active substances into living bodies in the medical field.

### Description of the Prior Art

Iontophoresis is a system of promoting transdermal or transmucosal absorption by use of electricity as an external stimulation. In the system, the transmittance of the molecules of a drug or medicine through a skin barrier is promoted on the basis of the principle that, in the electric field produced between the anode and the cathode by application of an electric current, positively charged molecules of the drug are moved from the anode toward the cathode and negatively charged molecules are moved from the cathode toward the anode [Journal of Controlled Release, Vol. 18, 213-220 (1992), Advanced Drug Delivery Review, Vol. 9, 119 (1992), and Pharmaceutical Research, Vol. 3, 318-326 (1986)].

Recent advances of synthetic technologies and genetic engineering lead to pure and mass production of naturally-occurring peptides or proteins, those peptides or proteins wherein their amino acid compositions are changed, or chemically modified derivatives thereof. The applications of these substances as drugs have now been expected. On the other hand, however, as studies on these peptides or proteins are in progress, it has been made clear that a diversity of biological activities of the peptides or proteins are physiologically controlled by the minute and complicated in vivo kinetics. In order to permit a peptide or protein to show the maximum medical efficacy on a specific disease and to suppress its side effect to a minimum, there has been required a system which enables one to strictly control the dosage of the peptide or protein. For instance, calcitonin has the capability of suppressing bone from being reduced in amount by suppression of the absorption of bone and is thus employed for the treatment of osteoporosis or Paget's disease. However, an excessive administration brings about a side effect such as inappetence. In addition, for the improvement of the therapeutic effect, it is essential to repeatedly administer the peptide or protein.

It is known that some types of peptides develop different medical efficacies depending on the manner of administration thereof. For instance, parathyroid hormone (or parathoromone) exhibits different actions which are contrary to each other, i.e. the bone absorption-promoting action and the bone formation-promoting action. When intravenously injected at a given rate, this hormone develops the bone resorption-promoting action, whereas the bone formation-promoting action is strongly developed for frequent administration through subcutaneous injection. In order to employ the parathyroid hormone as a drug for treating osteoporosis while expecting its bone formation-promoting action, the preparation thereof has to be of the pulse-release one, not of the sustained release one. However, it is known that such a biologically active peptide or protein is usually decomposed with a digestive fluid in the gastrointestinal tract, or is hydrolyzed with a hydrolase present in the digestive tract walls, thus being poor in absorptivity. This means that any satisfactory medical efficacy cannot be expected when these biologically active peptides or proteins are orally administered. Thus, usual practice is to administer them through injection. However, an injection causes a great pain to a patient and the self administration thereof is not possible, so that a great burden is imposed on the patient. This will become so much the worse for the case where repeated, continuous administration is required, for example, as in the case of the afore-indicated calcitonin or parathormone.

In the pharmaceutical or medical field, attention has now been paid to iontophoresis which is a novel drug delivery system responsible for the administration of such biologically active peptides or proteins, and extensive studies have been made thereon. More particularly, if drugs which have conventionally been able to be administered only as an injection can be developed as a preparation capable of being self-administered by patients themselves according to the iontophoresis, this will clear the way for treatment at home. The precise control in time of application of an electric current permits a desired absorption pattern to be created. Especially, in the supplemental therapy of an endogenous compound, it is conceivable to realize more effective therapy with the compound while taking the circadian rhythm of a living body into account.

Further, extensive studies have been made on applicators (which is used herein to broadly mean comprising devices such as interfaces, electrodes, accommodation containers and the like) to be employed for the iontophoresis, including an interface (a contact body to a skin or mucosa) for iontophoresis so as to have drugs transdermally absorbed in an efficient manner. For instance, there has been proposed an interface for iontophoresis which includes a drug retaining membrane designed to permit a drug to efficiently contact with a skin surface, and a drug solution internally capsuled in a spacer (Japanese Laid-open Patent Application No. 6-16535). However, this interface has limitations as to the efficient absorption of a water-soluble drug having a relatively great molecular weight through iontophoresis.

In order to solve the above problem, there has been proposed a technique of improving the absorptivity, for example, by applying absorption promoters (e.g. monoterpenes or aliphatic monoglycerides) showing surface activity to the inside of an interface (Japanese Laid-open Patent Application No. 6-16538).

EP 0 747 092 A2 relates to a composition for use in intophoresis comprising a drug and a humectant. A drug administration composition which comprises an ionizable drug and a humectant selected from the group consisting of a mixture of at least one amide and L-proline, wherein the at least one amide is an amide other than an acidic amino acid amine is not disclosed.

However, it has been usually accepted that absorption promoters act on a skin to bring about skin irritation, thus not ensuring the safety of their use. The interface for iontophoresis which has the drug retaining membrane and the drug solution on capsuled in the spacer is small in size and relatively high in absorptivity. This is disadvantageous in that the skin surface or the drug retainer is apt to be short of moisture or water as will be caused by the evaporation of the drug solution during the course of the application of an electric current, thereby lowering the electric conductivity of the interface. As a consequence, the use of this type of interface by long-time attachment disenables one to attain a good transdermal absorption through iontophoresis.

In view of the problems involved in the prior art, we paid attention to humectants which could prevent moisture or water from being evaporated from the inside of an interface for iontophoresis and act on a skin to show an absorption-promoting effect without bringing about any irritation against the skin. As a result of intensive studies and investigations on the humectants, we found that when a humectant is formulated in a drug solution on capsulated in an interface for iontophoresis or is incorporated in a drug retaining membrane, the interface is maintained over a long time as having the capability of being applied with an electric current therethrough or as having good electric conductivity. In addition, it was also found that when using such an interface, a drug could be transdermally administered reproducibly at very high bioavailability. The invention has been accomplished based on the above findings.

### SUMMARY OF THE INVENTION

It is accordingly an object of the invention to provide a drug administration composition for iontophoresis which comprises a humectant whereby the evaporation of water from a drug solution can be appropriately prevented at the time of the administration of a drug and the composition permits an electric current to be applicable therethrough over a long time.

It is another object of the invention to provide drug administration composition for iontophoresis wherein a humectant contained therein acts on keratin which is a barrier for delivery of a drug through a skin and promotes the transdermal absorption of the drug in response to an electric current being applied (wherein the drug is not absorbed when no electric current is passed, but is absorbed at the time of the application of an electric current) without damaging keratin, thus a high bioavailability of the drug being attained.

According to the invention, there is provided a drug administration composition for iontophoresis which comprises a drug to be ionized and a humectant selected from the group consisting of a mixture of at least one amide and L-proline, wherein the at least one amide is an amide other than an acidic amino acid amide. The composition of the invention is not only applicable to a skin, but also to mucosa.

### BRIEF DESCRIPTION OF THE DRAWINGS

Fig. 1 is a schematic sectional view showing an applicator of the invention;
Fig. 2 is a schematic sectional view showing another type of applicator;
Fig. 3 is a graph showing the concentration of hPTH (1→34) in serum in relation to the variation in time in Comparative Examples 1 - 3 and also in Comparative Examples 4 and 5;
Fig. 4 is a graph showing the concentration of hPTH (1→34) in serum in relation to the variation in time in Comparative Examples 6 - 8 and also in Comparative Example 9;
Fig. 5 is a graph showing the concentration of hPTH (1→34) in serum in relation to the variation in time at the time of its day-by-day iontophoretic administration in Example 1;
Fig. 6 is a graph showing the bioavailability of hPTH (1→34) in serum in relation to the variation in time at the time of its day-by-day iontophoretic administration in Example 1; and
Fig. 7 is a graph showing the concentration of hPTH (1→34) in serum in relation to the variation in time at the time of its iontophoretic administration in Examples 2-4.

### DETAILED DESCRIPTION OF THE INVENTION

The humectant used in the invention includes amides used singly or in combination. Of amides, it is more preferred to use urea, N-methyl-2-pyrrolidone or mixtures thereof. The humectant consists, in combination, of at least one amide and L-proline (hereinafter also referred to simply as co-humectant).

The drug to be ionized in the drug administration composition for iontophoresis of the invention include various types of drugs which are ionizable and have the capability of transdermal absorption, e.g. biologically active peptides or proteins having a molecular weight of 8000 or less or low molecular weight drugs. Examples of the biologically active peptides include calcium-regulating hormones, typical of which are those calcium-regulating hormones selected from parathormone, its derivatives and salts thereof. The drugs to be ionized may be either ones to be anionized or ones to be cationized. Preferably, those drugs to be cationized are used. If drugs to be cationized are used, it is preferred to cationize them in a buffer solution having a pH of 3 - 7. Such a buffer solution should preferably be one which contains a water-soluble aliphatic carboxylic acid. Examples of the water-soluble aliphatic carboxylic acid include aliphatic monocarboxylic acids, dicarboxylic acids and tricarboxylic acids having 2 to 6 carbon atoms which may preferably have 1 to 5 hydroxy group, and salts thereof. Examples of the aliphatic carboxylic acid include acetic acid, propionic acid, oxalic acid, ascorbic acid, lactic acid, succinic acid, citric acid, malic acid, tartaric acid, maleic acid, and salts and/or hydrates thereof. Of these, citric acid, succinic acid, tartaric acid, and salts and/or hydrates thereof are preferred. The salts preferably include inorganic salts such as sodium, calcium and potassium salts. More preferably, sodium salts are used.

The invention is now described in more detail. In the specification, drugs and biologically active substances may be sometimes referred to simply as "drug". It should be noted that where amino acids, peptides are expressed in terms of abbreviations in the present specification, the abbreviations used are those defined according to IUPAC-IUB Commission on Biochemical Nomenclature or those based on conventionally employed abbreviations. If there are optical isomers for amino acids, an amino acid used means an L isomer unless otherwise indicated.

The humectant contained in a drug solution of the invention may be any substance which can suppress moisture or water from being evaporated from the drug solution, keeps moisture on the surfaces of a skin and a mucosa or in a drug retainer, and acts on the keratin of a skin and a mucosa without adversely influencing a skin or mucosa. Examples include amides. Examples of the amide include urea, dimethylacetamide, diethyltoluamide, dimethylformamide, dimethyloctamide, dimethyldecamide, biodecomposable urea (1-alkyl-4-imidazolin-2-on), N-methyl-2-pyrrolidone, 2-pyrrolidone, 1-lauryl-2-pyrrolidone, 1-methyl-4-carboxy-2-pyrrolidone, 1-hexyl-4-carbonyl-2-pyrrolidone, 1-lauryl-4-carboxy-2-pyrrolidone, 1-hexyl-4-methoxycarbonyl-2-pyrrolidone, 1-lauryl-4-methoxycarbonyl-2-pyrrolidone, N-cyclohexylpyrrolidone, N-dimethylaminopropylpyrrolidone, N-cocoalkylpyrrolidone, tallow alkylpyrrolidone, N-(2-hydroxyethyl)-2-pyrrolidone, 1-dodecylazacycloheptan-2-on, 1-geranylazacycloheptan-2-on, 1-farnesylazacycloheptan-2-on, 1-(3,7-dimethyloctyl)azacycloheptan-2-on, 1-(3,7,11-trimethyldodecyl)azacycloheptan-2-on, 1-geranylazacyclohexan-2-on, 1-geranylazacycloheptan-2,5-dion, 1-farnesylazacyclopentan-2-on, 1-[2-(decylthio)ethyl]azacyclopentan-2-on, and the like. Of these, urea and N-methyl-2-pyrrolidone are preferred. The use of urea and N-methyl-2-pyrrolidone in combination is more effective.

In the practice of the invention as the humectant, at least one amide humectant and a co-humecant, L-proline, wherein the at least one amide is an amide other than an acidic amino acid amide are used in combination. L-proline is very effective in reducing the skin irritation at the time of iontophoretic administration and is most preferably used in combination with urea, N-methyl-2-pyrrolidone or mixtures thereof.

The content of a humectant of the invention in a drug solution is appropriately selected from a range of amount which is sufficient to suppress the evaporation of moisture from the drug solution and keep moisture on the skin or mucos surface and in a drug retainer. More particularly, the content is in the range of 0.1 to 90% (w/w), preferably 0.5 to 80% (w/w) and more preferably 1 to 30% (w/w). This is true of the case where an amide is used in combination with a co-humectant. The ratio between the amide humectant and the co-humectant may be appropriately selected from a range amount within which the purpose of the invention is achievable. For instance, where urea, N-methyl-2-pyrrolidine or a mixture thereof is used as a humectant, the content in the composition may be, for example, in the range of 1 to 30% (w/w). Alternatively, where a mixture of urea and N-methyl-2-pyrrolidone is used, the ratio by weight is in the range of 1:30 to 30:1, preferably 1:10 to 10:1, and more preferably about 1:1. L-proline is used as the co-humectant, in a content of above 1 to 30% (w/w) of the composition.

According to a preferred embodiment of the invention, there is provided a drug administration composition for iontophoresis which comprises (i) a drug to. be ionized, and (ii) a humectant consisting of a member selected from a group consisting of (a) N-methyl-2-pyrrolidone and L-proline, (b) urea, N-methyl-2-pyrrolidone and L-proline, and (c) urea and L-proline. Among these, the humectant preferably consists of (c) urea and L-proline.

These compositions may further comprise a water-soluble aliphatic carboxylic acid having from 2 to 6 carbon atoms. Preferred examples of the acid include citric acid, salts and/or hydrates thereof. In this case, the carboxylic acid is formulated in such a way that the ratio between the molecular amount of a drug to be ionized and the molar equivalents of an aliphatic carboxylic acid (i.e. the molecular amount of the carboxylic acid × the numerical value of the electric charge of the carboxylic acid) is in the range of 1:20 to 1:100,000, preferably 1:40 to 1:50,000. If the humectant used consists of (c) urea and L-proline, the ratio by weight between urea and L-proline is in the range of 30:1 to 1:30, preferably 5:1 to 1:5, more preferably 1:2.

The drug holder or retainer which constitutes an interface for iontophoresis may be made of various types of members which are capable of retaining a drug therewith and which have a porous or capillary structure through which a dissolved drug is permeable (the member having a porous or capillary structure being sometimes referred to simply as a porous member or body in the present specification). A preferred porous body includes an organic one. Examples of the organic porous body include fibrous bodies or masses made of naturally occurring fibers such as cellulose, semi-synthetic fibers such as cellulose acetate, and synthetic fibers such as polyethylene, polypropylene, nylons, polyesters and the like, sheets such as of paper, woven or non-woven fabrics, and porous synthetic resins such as porous polypropylene, porous polystyrene, porous polymethyl methacrylate, porous nylons, porous polysulfones, porous fluorine resins, fluorinated polyvinylidene introduced with a hydrophilic group. Especially, the fluorinated polyvinylidene introduced with a hydrophilic group is preferred because this resin is low in adsorption of a drug such as a peptide and is able to readily release it on contact with water.

The porous body is not critical with respect to its shape and may be appropriate used in the form of a sheet or the like. When a porous body is in the form of a sheet, its thickness is appropriately selected depending on the amount of a drug to be retained and is, for example, in the range of 1 to 500 µm, preferably 10 to 200 µm. The porous body may be soft or plastic, or may be non-deformable. The area of the sheet-shaped porous body is appropriately selected depending on the amount of a drug to be retained and the area of a skin or mucosa to be contacted. For example, the area is in the range of 1 to 20 cm², preferably from 2 to 10 cm². The pore size of the sheet-shaped porous body is conveniently selected within a range not impeding the retaining amount and the releasing property of a drug. For example, an average pore size is in the range of 0.01 to 50 µm, preferably from 0.2 to 20 µm.

The drugs retained in the porous body in the practice of the invention include various types of drugs which are ionizable and are transdermally absorbed and include, for example, biologically active peptides or proteins whose molecular weight is 8000 or less, or low molecular weight drugs. As for the ionization, it is sufficient that at least a part of a drug being retained is ionized. The ionized drug may be an anionized one or a cationized one. Preferably, drugs to be cationized are used. Biologically active peptides include, for example, those peptides shown in (1) to (9) below, of which peptides having a molecular weight of 5000 or less are preferably used.
(1) 1) Luteinizing hormone-releasing hormone (LH-RH), 2) derivatives thereof (herein "derivatives thereof' of proteins and peptides include their analog, muteins fractions and the structurally similar peptide which have similar function or activities) having such an activity as LH-RH, and 3) polypeptides represented, for example, by the following formula (I) or salts thereof (United States Patent Nos. 3853837, 4008209 and 3972859, British Patent No. 1423083, and Proceedings of the National Academy of Science, Vol. 78, pp. 6509 to 6512 (1981)) (Pyr)Glu-R1-Trp-Ser-R2-R3-R4-Arg-Pro-R5 ..... (I) wherein R1 represents His, Tyr, Trp or p-NH₂-Phe, R2 represents Tyr or Phe, R3 represents Gly or a D-amino acid residue, R4 represent Leu, Ile or Nle, and R5 represents Gly-NH-R6 in which R6 represents hydrogen atom or a lower alkyl group which may have a hydroxyl group or NH-R6 in which R6 is as defined above.
(2) 1) LH-RH antagonists, and 2) polypeptides represented, for example, by the following formula (II) or salts thereof (United States Patent Nos. 4086219, 4124577, 4253997 and 4317815) 
N-α-t-butoxycarbonyl-O-benzyl-Ser-Trp-Ser-Tyr-X1-Leu-Arg-Pro-
GlyNH2 ..... (II)
 wherein X1 represents D-Ser or D-Trp.
(3) 1) Insulin, 2) somatostatin, 3) somatostatin derivatives, and 4) polypeptides represented, for example, by the following formula (III) or salts thereof (United States Patent Nos. 4087390, 4093574, 4100117 and 4253998) 
H-L-Ala-Y-L-Cys-L-Lys-Z-L-Phe-L-Phe-D-Trp-L-Lys-L-
..... (III)
Thr-L-Phe-L-Thr-L-Ser-L-Cys-OH
 wherein Y represents D-Ala, D-Ser- or D-Val, and Z represents Asn or Ala.
(4) 1) Adrenocorticotropic hormone (ACTH), 2) melanocyte-stimulating hormone (MSH), 3) thyroid stimulating hormone releasing hormone (TRH) and derivatives thereof, and 4) compounds represented, for example, by the following formula (IV) or salts thereof (Japanese Laid-open Patent Application Nos. 50-121273 and 52-116465) 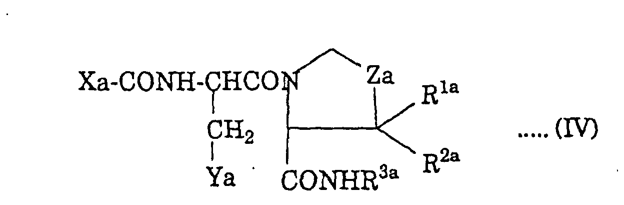 wherein Xa represents a 4, 5 or 6-membered heterocyclic group, Ya represents imidazol-4-yl or 4-hydroxyphenyl, Za represents CH₂ or S, R^{1a} and R^{2a} may be the same or different and represent hydrogen atom or a lower alkyl group, and R^{3a} represents hydrogen atom or an optionally substituted aralkyl group.
(5) Parathyroid hormone (PTH) and derivatives thereof, including 1) peptides represented, for example, by the following formula (V) or salts thereof (Japanese Laid-open Patent Application Nos. 5-32696 and 4-247034, and European Patent Laid-open Application Nos. 510662, 477885 and 539491), 2) a peptide fragment terminated with N of human PTH (1→34 position) (hereinafter referred to simply as hPTH (1→34)) [G. W. Tregear et al., Endocrinology, 93, 1349-1353 (1973)], and 3) vasopressin and vasopressin derivatives (e.g. desmopressin [Journal of Society of Endocrinology, Japan, Vol. 54, No. 5, pp. 676-691 (1978)]}. The diseases to be applied through the iontophoretic administration of the above-mentioned PTH and derivatives, peptides of formula (V) or salts thereof, and hPTH (1→34) include bone disease such as osteoporosis, and fractures, myocardial infarction. 
R1'-Val-Ser-Glu-Leu-R2'-His-Asn-R3'-R4'-R5'-His-Leu-Asn-Ser-R6'-R7'-
Arg-R8'-Glu-R9'-Leu-R10'-R11'-R12'-Leu-Gln-Asp-Val-His-Asn-R13'
..... (V)
 wherein R1' represents Ser or Aib, R2' represents Met or a naturally-occurring lipophilic amino acid residue, R3' represents Leu, Ser, Lys or an aromatic amino acid residue, R4' represents Gly or a D-amino acid residue, R5' represents Lys or Leu, R6' represents Met or a naturally occurring lipophilic amino acid residue, R7' represents Glu or a basic amino acid residue, R8' represents Val or a basic amino acid residue, R9' represents Trp or 2-(1,3-dithiolan-2-yl)Trp, R10' represents Arg or His, R11' represents Lys or His, R12' represents Lys, Gln or Leu, and R13' represents Phe or Phe-NH₂.
(6) 1) Oxytocin, 2) calcitonin, 3) derivatives having a function similar to calcitonin, 4) compounds represented, for example, by the following formula (VI) or salts thereof [Endocrinology, 1992, 131/6 (2885-2890)], 5) glucagon, 6) gastrin, 7) secretin, 8) cholecystokinin and 9) angiotensin 
Cys-Ser-Asn-Leu-Ser-Thr-X^b-Val-Leu-Gly-Lys-Leu-Ser-Gln-Glu-Leu-His-
Lys-Leu-Gln-Thr-Tyr-Pro-Arg-Thr-Asp-Val-Gly-Ala-Gly-Thr-Pro ..... (VI)
 wherein Xb represents 2-aminosberic acid residue
(7) 1) Enkephalin, 2) derivatives of enkephalin, and 3) oligopeptides and endorphins such as peptides represented, for example, by the following formula (VII), or salts thereof (United Stated Patent No. 4277394 and European Laid-open Patent Application No. 31567) 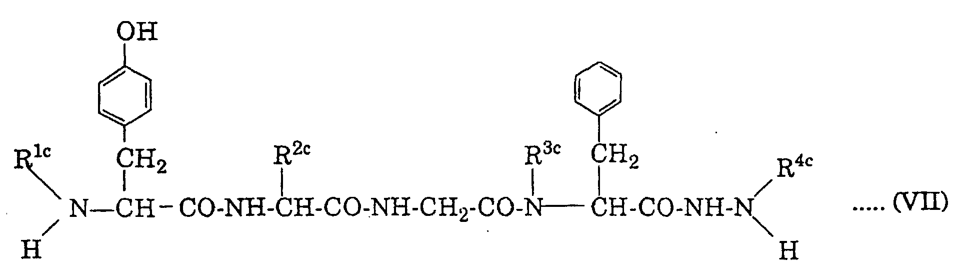 wherein R^{1c} and R^{3c} independently represent hydrogen atom or an alkyl group having 1 to 6 carbon atoms, R^{2c} represents hydrogen atom or a residue of D-α-amino acid, R^{4c} represents hydrogen atom or an optionally substituted aliphatic acyl group having 1 to 8 carbon atoms.
8) 1) Kyotorphine, 2) interleukins (I to XI), 3) tuftsin, 4) thymopoietin, 5) thymus humoral factor (THF), 6) factor of thymus in serum (FTS) and derivatives thereof, 7) peptides represented by the following formula (VIII), or salts thereof (United States Patent No. 4229438), and 8) other thymus hormones [ e.g. thymocins α₁ and β₄, thymic factor X and the like, "Journal of Clinical Experimental Medicine (Igakuno Ayumi)", Vol. 125, No. 10, pp. 835-843 (1983)] PGlu-Xd-Lys-Ser-Gln-Yd-Zd-Ser-Asn-OH ..... (VIII) wherein Xd represents L-Ala or D-Ala, Yd and Zd independently represent Gly or a D-amino acid residue having 3 to 9 carbon atoms.
(9) a) Motilin, b) dynorphin, c) bombesin, d) neurotensin, e) cerulein, f) bradykinin, g) urokinase, h) substance P, i) polymyxin B, j) colistin, k) gramicidin, l) bacitracin, m) protein synthesis-stimulating peptide, n) gastric inhibitory polypeptide (GIP), o) vasoactive intestinal polypeptide (VIP), p) platelet-derived growth factor (PDGF), and growth hormone-releasing factor (GRF, somatoclinine).

These biologically active peptides may be human peptides, or peptides derived from other animals such as bovines, swine, chickens, salmon, eel and the like, along with chimeras of the human peptides and the peptides derived from other animals. Further, active derivatives wherein the structures of the peptides are partially modified may also be used. For instance, there may be used insulin derived from swine, calcitonins derived, for example, from swine, chicken, salmon and eel, or a peptide which consists of a human and salmon chimera and represented by the following formula (IX) [Endocrinology, 1992, 131/6, (2885-2890)].

Cys-Gly-Asn-Leu-Ser-Thr-Cys-Met-Leu-Gly-Lys-Leu-Ser-Gln-Glu-
Leu-His-Lys-Leu-Gln-Thr-Tyr-Pro-Arg-Thr-Asn-Thr-Gly-Ser-Gly-
Thr-Pro ..... (IX)

The low molecular weight drugs used in the practice of the invention include compounds whose molecular weight is about 1000 or less and having the pharmacological activity. The low molecular weight drugs are not critical with respect to the type and include, for example, antibiotics, antimycotic drugs, hypolipidermic drugs, circulatory drugs, antiplatelet drugs, antitumor drugs, antipyretic, analgesic and/or anti-inflammatory agents, antitussive-expectorant agents, sedatives, muscle relaxtants, antiepileptic drugs, antiulcer drugs, antidepressant agents, antiallergic agents, cordiotoncis, vasodilators, hypotensive-diuretic agents, antiarrhythmic agents, drugs for diabetes, anticoagulants, hemostatic agents, antituberculotic drugs, hormones, narcotic antagonists, bone resorption-inhibitory agents, osteogenetic promoter, angiogenesis inhibitors, and local anaesthetic agents.

Examples of the antibiotic include gentamycin, lividomycin, sisomycin, tetracycline hydrochloride, ampicillin, cefalothin, cefotiam, cefazolin, tienamycin, sulfazecin and the like. Examples of the antimycotic agent include 2-[(1R, 2R)-2-(2,4-difluorophenyl-2-hydroxy-1-methyl-3-( 1H-1,2,4-triazol-1-yl)propyl]-4-[4-(2,2,3,3-tetrafluoropropoxy)phenyl]-3-(2H, 4H)-1,2,4-triazolone. Examples of the hypolipidermic include paravastatin, simvastatin. Examples of the circulatory drug include delapril hydrochloride.

Examples of the antiplatelet drug include ticlopidine, cilostazol, limaprostat, aspirin. Examples of the antitumor drug include bleomycin hydrochloride, actinomycin D, mitomycin C, adriamycin, fluorouracil and the like. Examples of the antipyretic, analgesic and/or anti-inflammatory agents include sodium salicylate, sulpyrine, indomethacin sodium, dichlorophenac sodium, buprenorphine hydrochloride, pentazocine, eptazocine hydrobromide, butorphanol tartarate, tramadole hydrochloride, ketrolac, meperidine hydrochloride, morphine hydrochloride, morphine sulfate, hydromorphone (hydromorphine), fentanyl citrate, fentanyl, sufentanil, alfentanil and the like. Examples of the antitussive and expectorant agent include ephedrine hydrochloride, codeine phosphate, picoperidamine hydrochloride. Examples of the sedative include chloropromazine hydrochloride, atropine sulfate. Examples of the local anaesthetic agents include Lidocaine. Examples of the muscle relaxant include pridinol methanesulfonate, tubocurarine chloride. Examples of the antiepileptic agent include phenytoin sodium, ethosuximide.

Examples of the antiulcer drug include metoclopramide. Examples of the antidepressant include imipramine, phenelzine sulfate. Examples of the antiallergic drug include diphenylhydramine hydrochloride, tripelennamine hydrochloride, clemizole hydrochloride. Examples of the cardiotinic include trans-π-oxocamphor, theophyllol. Examples of the antiarrhythymic agent include propranolol hydrochloride, oxyprenolol hydrochloride. Examples of the vasodilator include oxyfedrine hydrochloride, tolazoline hydrochloride, bamethan sulfate. Examples of the hypotensive-diuretic agent include pentolinium, hexamethonium bromide. Examples of the antidiabetic agent include glymidime sodium, glypizide, metformin. Examples of the anti-coagulant include sodium citrate.

Moreover, examples of the hemostatic include menadione sodium bisulfite, acetomenaphtone, tranexiamic acid. Examples of the antituberculosis drug include isoniazid, ethambutol. Examples of the hormone drug include β-estradiol, testosterone, predonisolone succinate, dexamethasone sodium sulfate, methimazole. Examples of the narcotic antigonist include levalorphan tartarate, nalorphine hydrochloride. Examples of the bone resoption inhibitors include (sulfur-containing alkyl)aminomethylene bisphophoate. Examples of the angiogenesis inhibitor include a vascularization inhibitory steroid [Science, Vol. 221, p. 719 (1983)], and fumagillol derivatives [e.g. O-monochloroacetyl-carbamoylfumagillol, O-dichloroacetylcarbomoylfumagillol (European Laid-open Patent Application Nos. 357061, 359036, 386667 and 415294).

These drugs may be retained by dissolving them, for example, in distilled water for injection or physiologically saline for injection and applying the resultant solution to the drug-retaining layer according to an ordinarily employed procedure including, for example, impregnation, spraying or dipping and drying. Where a physiologically active peptide is used as a drug, a disaccharide (e.g. trehalose, maltose, mannitol, inositol ) may be added to the drug solution in an amount, for example, of 1 to 100 mg/ml. The addition of a disaccharide can improve the stability of the drug in a dry condition. When the drug retainer in which a drug has been retained is preserved in a dry condition, it can be preserved over a long peri od of time while keeping the activity of the drug. More particularly, after a drug retainer retaining a drug therein has been well dried, the retainer may be packed, according to a method such as of vacuum seal packaging, in a film (e.g. an aluminium foil or film) whose moisture permeability is very low. In order to ensure the dry conditions, a drying agent [e.g. a zeolite drying agent such as Ceramu (Registered Trade Name of Tokai Chemical Co., Ltd.) or a silica gel drying agent] may be incorporated in the package when the vacuum seal packaging is performed. If a drug undergoes oxidative decomposition, an oxygen absorbing agent [e.g. Ageless (Registered Trade Name of Mitsubishi Gas Chemical Co., Ltd.)] may further be incorporated along with the drying agent.

The drug should be present in the drug-retaining layer in an effective amount which depends on the type of drug, the type of drug-retaining layer and the site to be administered. For instance, the amount is in the range of from 0.1 to 50000 µg, preferably from 0.5 to 1000 µg/cm² of the drug-retaining layer. It is not always necessary to retain a drug in a thin membrane, but a drug solution may be injected or charged in the vicinity of the thin membrane when used. The liquid for dissolving a drug may contain an appropriate type of adsorption inhibitor for inhibiting the loss of biologically active peptides and proteins due to adsorption [e.g. bovine serum albumin (BSA), human serum albumin, benzalkonium chloride, benzethonium chloride, Tween 80].

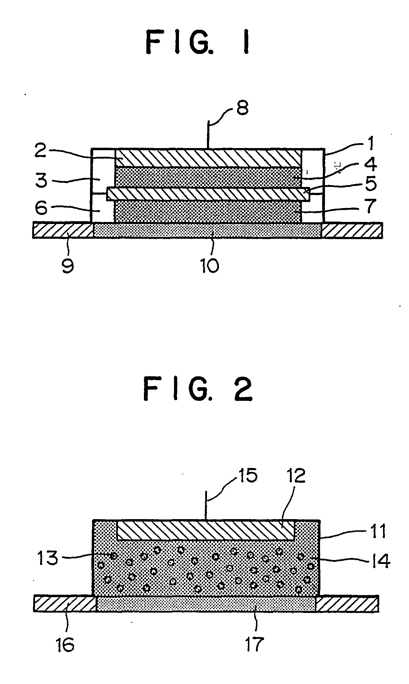

An applicator which is provided with an interface described hereinafter is useful for transdermal administration of a drug through iontophoresis. Reference is now made to Figs. 1 and 2 which are, respectively, a sectional view showing an applicator having such an interface as mentioned above. Fig. 1 shows an ion exchange membrane type applicator, and Fig. 2 shows an ion exchange resin type applicator. It will be noted that materials and sizes of individual members or parts disclosed below are indicated only for convenience's sake and are not limitative.

Fig. 1 shows a fundamental structure of an applicator for iontophoresis which includes a cup-shaped container 1 (made, for examples of a polyester and having an inner diameter of 30 mm) having a cylindrically recessed portion. The container 1 has, at the bottom thereof, an electrode 2 (consisting, for example, of a silver foil electrode punched out in the form of a circle with a diameter of 23 mm and a thickness of 0.04 mm), a ring 3 (made, for example, of polyethylene in the form of an O ring with an inner diameter of 23 mm and an outer diameter of 28 mm), a conductive layer 4 (made, for example, of a non-woven fabric commercially available from Japan Vilene Co., Ltd., under the designation of WP-2085 and having a diameter of 23 mm and a thickness of 0.63 mm), an ion exchange membrane 5 (such as, for example, AC201 membrane commercially available from Asahi Chemical Industries, Ltd., with a diameter of 28 mm and a thickness of 0.23 mm), a ring 6 (made, for example, of polyethylene in the form of an O ring having an inner diameter of 23 mm and an outer diameter of 28 mm), and a conductive layer 7 (made, for example, of a non-woven fabric commercially available from Japan Vilene Co., Ltd., under the designation of LMW-9007 and having a diameter of 23 mm and a thickness of 0.63 mm), arranged in this order as shown.

The cup-shaped container 1 is so designed as to have such a thickness that the lower face of the container 1 is at the same level as one surface of the conductive layer 7. The cup-shaped container 1 has a small hole at the center of the bottom thereof so that the electrode 2 is connected with an electric supply via a connection terminal 8 (i.e. a connector to the electric supply) attached to the small hole. The thus constituted interface is attached with a drug retaining membrane 10 (a thin membrane retaining a drug therein, e.g. Hydrophilic Durapore, Registered Trade Name of Millipore Co., Ltd., with a diameter of 28 mm and a thickness of 0.125 mm) which has been preliminarily attached to a self-adhesive layer 9 (e.g. adhesive sheet having hole with a diameter of 23 mm, Blenderm made by 3M Pharmaceuticals M.N), thereby permitting the drug-retaining membrane 10 to be brought into contact with a skin on application of the interface. The humectant and buffer solution components of the invention are applied to the conductive layer 4, the ion exchange membrane 5 and/or the drug-retaining layer 10.

In Fig. 2, there are shown a cup-shaped container 11 having a cylindrically recessed portion (made, for example, of polypropylene with an inner diameter of 30 mm), an electrode 12 (consisting, for example, of a silver foil electrode punched out in the form of a circle with a diameter of 23 mm and a thickness of 0.04 mm), and a gel layer 14 (e.g. an agar gel layer commercially available from Ina Foods Co., Ltd., under the designation of UP-16) having, for example, an ion exchange resin 13 containing 8 wt% of cholestyramine (a strongly basic anion exchange resin containing a quaternary ammonium salt and a chloride ion and manufactured by Sigma Co., Ltd.) dispersed and/or mixed therein. The cup-shaped container 11 is so designed as to have a thickness which allows the lower face of the container 11 is at the same level as the surface of the gel layer 14.

The gels used as the gel layer are those materials which can disperse anion exchange resin therein and are able to dissolve an effective component in the drug layer when the interface is used. Preferably hydrophilic, polymeric gels are used. Examples of such gels include agar, gelatin, xanthan gum, locust bean gum, carrageenan, gellam gum, tamarind gum, curdlan, pectin, furcellaran, guar gum, alginic acid, sodium alginate, tara gum, karaya gum, cellulose and derivatives thereof. These may be used singly or in combination. If necessary, the gel layer may further comprise electrolytes, pH adjusters, stabilizers, thickeners, wetting agents, surfactants, dissolution aids, absorption promoters, preservatives and the like.

The electrode 12 has a connection terminal 15 ( a connector to an electric supply) extending from a small hole which is made at the center of the cup, so that the electrode 12 can be connected with the electric supply. The thus constituted interface is attached with a drug-retaining layer 17 (a thin membrane retaining a drug therein, e.g. Hydrophilic Durapore Membrane with a diameter of 28 mm and a thickness of 0.125 mm) which has been preliminarily attached to a self-adhesive layer 16 (e.g. a self-adhesive sheet, Blenderm made by 3M Pharmaceuticals. M.N.) having a hole with a diameter of 23 mm, thereby permitting the drug-retaining membrane 17 to be brought into contact with a skin on application of the interface. The humectant and buffer solution components of the invention are applied to the gel layer 14 and/or the drug-retaining layer 17.

The transdermal administration of a drug through iontophoresis can be effected by applying a DC voltage between the electrode of the interface and a reference electrode to pass an electric current thereacross. The electric supply may be one which is capable of applying a continuous DC voltage or a pulse DC voltage. Preferably, an electric supply capable of applying a pulse DC voltage is used. The power supply for the pulse DC voltage should preferably be one which is able to apply a square pulse DC voltage. The frequency of the pulse DC voltage should preferably be selected from a range of from 0.1 to 200 kHz, more preferably from 1 to 100 kHz, and most preferably from 5 to 80 kHz. The on/off ratio of the pulse DC voltage is selected from a range of from 1/100 to 20/1, preferably from 1/50 to 15/1 and more preferably from 1/30 to 10/1. The application time for continuous application is 24 hours or less, preferably 12 hours or less, and more preferably 6 hours or less. For intermittent application, the application time is 24 hours or less, preferably 12 hours or less and more preferably 6 hours or less, each in total time.

The invention is more particularly described by way of examples which should not be construed as limiting the invention thereto. Comparative examples are also described.

### Experimental Example 1

This Experimental Example 1 was conducted using an applicator having an interface shown in Fig. 1. The materials and sizes of the individual members of the interface were, respectively, those which were particularly described with reference to Fig. 1 hereinbefore. Male SD rats (seven weeks old) were clipped with a hair clipper at an abdominal skin thereof and then treated with a shaver under anesthetization with pentobarbital, followed by slightly rubbing with an absorbent cotton containing a 70% aqueous solution of ethanol for the purpose of defatting and disinfection. An applicator for a drug was attached to the abdominal skin of the rat at the left side thereof with respect to the median line, and a 12% PVA (polyvinyl alcohol) gel (containing 0.9% of sodium chloride and having a thickness of 2 mm) was attached to the left side abdominal skin of the rat as a reference electrode (cathode). A silver electrode was fixed to the applicator, and a silver chloride electrode (2.5 cm²) was fixed as the reference electrode.

In the iontophoresis, a pulse depolarization direct current (frequency = 30 kHz, on/off = 3/7, and voltage = 10 V) caused by a short-circuiting switch was used, and a current application cycle was repeated three times wherein the current was applied to for 15 minutes and was stopped for 5 minutes, followed by repeating the current application and stopping procedure three times (i.e. a total time of the current application and stopping was 60 minutes with an actual application time of 45 minutes) and then allowing to stand for 60 minutes under conditions where no electric current was passed. During the course of repetition of the cycle, the rat was fixedly kept under anesthetization with pentobarbital. The concentration of a peptide in serum was determined according to the radioimmunoassay method using Rat PTH Kit (Trade Name: Nicols Co., Ltd.). In the interface shown in Fig. 1, the following solution compositions of the respective examples and the comparative examples were each impregnated in the non-woven fabric 7 used as a solution for electric conductor layer. The experiment was conducted in such a way that 40 µ g of hPTH (1→34) used a drug was contained in the drug retaining membrane 10. The contents of all components are by weight.

### Comparative Example 1

| Components | Content (%) |
|---|---|
| Urea | 5 |
| Citrate buffer solution (33 mM, pH = 5) | 95 |
| Total | 100 |

### Comparative Example 2

| Components | Content (%) |
|---|---|
| N-Methyl-2-pyrrolidone | 5 |
| Citrate buffer solution (33 mM, pH = 5) | 95 |
| Total | 100 |

### Comparative Example 3

| Components | Content (%) |
|---|---|
| Urea | 5 |
| N-Methyl-2-pyrrolidone | 5 |
| Citrate buffer solution (33 mM, pH = 5) | 90 |
| Total | 100 |

### Comparative Example 4

| Component | Content (%) |
|---|---|
| Citrate buffer solution (33 mM, pH = 5) | 100 |
| Total | 100 |

### Comparative Example 5

| Components | Content (%) |
|---|---|
| Propylene glycol | 30 |
| Citrate buffer solution (33 mM, pH = 5) | 70 |
| Total | 100 |

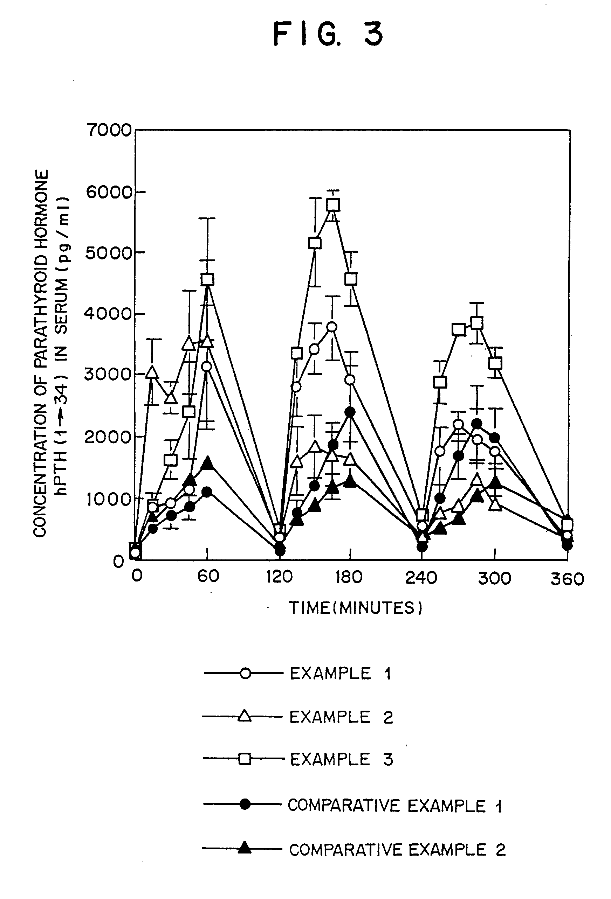

The results obtained in Comparative Examples 1 to 5 are shown in Fig. 3 and Table 1.

It will be appreciated from Fig. 3 that in Comparative Examples 1 to 3, higher concentrations in serum are reproducibly obtained than in Comparative Examples 4, 5. With the case of Comparative Example 3 (indicated by symbol "□" in Fig. 3) wherein urea and N-methyl-2-pyrrolidone are used in combination as a humectant, the concentration is more improved over those of Comparative Example 1 (urea: indicated by symbol "○" in Fig. 3) where urea is singly used and Comparative Example 2 (N-methyl-2-pyrrolidone: indicated by symbol "Δ" in Fig. 3) where N-methyl-2-pyrrolidone is singly used. Especially, with Comparative Example 3, the concentration of parathyroid hormone, hPTH (1→34), in serum reached a level as high as 6000 pg/ml at the second cycle and 4000 pg/ml at the third cycle. Table 1 shows a bioavailability (B. A.) which was calculated from an area under peptide-in-serum concentration-time curve (AUC) obtained by intravenous administration of hPTH (1→34) at a dose of 2 µg/kg. As shown in Table 1, the bioavailabilities of Comparative Examples 1 to 3 are much better than those of Comparative Examples 4, 5. Especially, with Comparative Example 3, the bioavailability (B. A.) is as high as 48.89%.

**Table 1**

| Comparative Example | Bioavailability B. A. % | Standard Error (SE) |
|---|---|---|
| Example 1 | 32.81 | 5.68 |
| Example 2 | 26.81 | 6.60 |
| Example 3 | 48.89 | 5.11 |
| Example 4 | 17.35 | 2.88 |
| Example 5 | 14.83 | 1.54 |

### Experimental Example 2

In the Experimental Example 2, the experimental procedure was conducted substantially in the same manner as in Experimental Example 1 wherein an applicator used was as shown in Fig. 2. The constituent materials and sizes of the respective members were those particularly described hereinbefore with reference to Fig. 2. In the interface shown in Fig. 2, the agar gel layer 14 was, respectively, impregnated with the following solution compositions of the Comparative Examples to provide a solution for an electric conductor layer. The drug which consisted of 40 µg of hPTH (1→34) was impregnated in the drug retaining membrane 17. The contents of individual components are by weight.

### Comparative Example 6

| Components | Content (%) |
|---|---|
| Urea | 5 |
| Agar | 1 |
| Cholestyramine | 8 |
| Citric acid monohydrate | 0.25 |
| Trisodium citrate dihydrate | 0.7 |
| Distilled water for injection | 85.05 |
| Total | 100 |

### Comparative Example 7

| Components | Content (%) |
|---|---|
| N-Methyl-2-pyrrolidone | 5 |
| Agar | 1 |
| Cholestyramine | 8 |
| Citric acid monohydrate | 0.25 |
| Trisodium citrate dihydrate | 0.7 |
| Distilled water for injection | 85.05 |
| Total | 100 |

### Comparative Example 8

| Components | Content (%) |
|---|---|
| Urea | 5 |
| N-Methyl-2-pyrrolidone | 5 |
| Agar | 1 |
| Cholestyramine | 8 |
| Citric acid monohydrate | 0.25 |
| Trisodium citrate dihydrate | 0.7 |
| Distilled water for injection | 80.05 |
| Total | 100 |

### Comparative Example 9

| Components | Content (%) |
|---|---|
| Agar | 1 |
| Cholestyramine | 8 |
| Citric acid monohydrate | 0.25 |
| Trisodium citrate dihydrate | 0.7 |
| Distilled water for injection | 90.05 |
| Total | 100 |

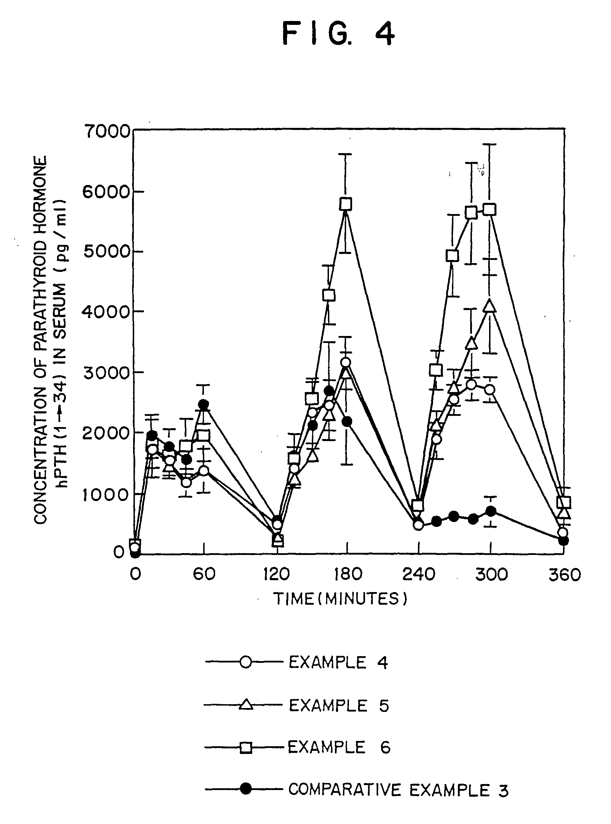

The results of Comparative Examples 6 to 9 are shown in Fig. 4 and Table 2. It will be appreciated from Fig. 4 that in Comparative Examples 6 to 8, higher peptide concentrations in serum are reproducibly obtained than in Comparative Example 9. With the case of Comparative Example 8 (indicated by symbol "□" in Fig. 4) wherein urea and N-methyl-2-pyrrolidone are used in combination as, a humectant, the concentration is more improved over those of Comparative Example 6 (urea: indicated by symbol "○" in Fig. 4) where urea is singly used and Comparative Example 7 (N-methyl-2-pyrrolidone: indicated by symbol "Δ" in Fig. 4) where N-methyl-2-pyrrolidone is singly used. Especially, with Comparative Example 8, the concentration of hPTH (1→34), in serum reached a level as high as 5900 pg/ml at the second cycle and 5800 pg/ml at the third cycle. Table 2 shows a bioavailability (B. A.) which was calculated from an area under peptide-in-serum concentration-time curve (AUC) obtained by intravenous administration of hPTH (1→34) in an amount of 2 µg/kg. As shown in Table 2, the bioavailabilities of Comparative Examples 6 to 8 are much better than that of Comparative Example 9. Especially, with Comparative Example 8, the bioavailability (B. A.) reached a level as high as 54.85%.

**Table 2**

| Comparative Example | Bioavailability (B. A.) % | Standard Error (SE) |
|---|---|---|
| Example 6 | 32.17 | 3.56 |
| Example 7 | 35.27 | 4.31 |
| Example 8 | 54.85 | 5.89 |
| Example 9 | 18.35 | 2.06 |

### Experimental Example 3

In this Experimental Example 3, an applicator of the type shown in Fig. 1 was used. The materials and sizes of the applicator were those having set out with reference to Fig. 1. In the applicator shown in Fig. 1, the non-woven fabrics 4, 7 were used and a solution for electric conductor layer was impregnated in the ion exchange membrane 5. Using this applicator, rats under non-anesthetization were subjected to iontophoretic administration day by day to check a variation in absorptivity. The administration test of the rats was carried out under substantially the same conditions as in Experimental Example 1 except that the manner of continuous administration set out below was adopted. More particularly, rats were each shaved under anesthetization with ether at the left half of the abdominal portion (including a nearby back portion) with respect to the median line, on which a drug-applied applicator was attached to the side of the abdominal portion. After continuous administration over five consecutive days, the administration was stopped for two days, followed by consecutive five-days continuous administration at the left half of the abdominal portion, allowing a non-administered period of two days, and further continuous administration over 2 days. Upon the administration, care was paid so that the attached patch was not removed from the abdominal portion, for which the abdominal portion was wound with a stretchable bandage and the rat was retained with the Bollman - cage.

It should be noted that prior to this experiment, such an applicator as used in Experimental Example 1 was used for pretest wherein the iontophoretic administration was repeated over 3 days and the administered sites of skin were observed, revealing that symptoms of skin irritation caused by application of an electric current were observed although slight in all cases. To avoid this, the following composition wherein 10% of L-proline was formulated in the composition of Comparative Example 1 was used, from which it was found that skin irritation could be avoided. According to this finding, a solution for electric conductor layer which had a composition in Example 1 was used, and 200 µg of hPTH (1→34) was contained in the drug retaining membrane 10. The contents of individual components are by weight.

### Example 1

| Components | Content (%) |
|---|---|
| Urea | 5 |
| L-Proline | 10 |
| Citrate buffer solution (33 mM, pH = 5) | 85 |
| Total | 100 |

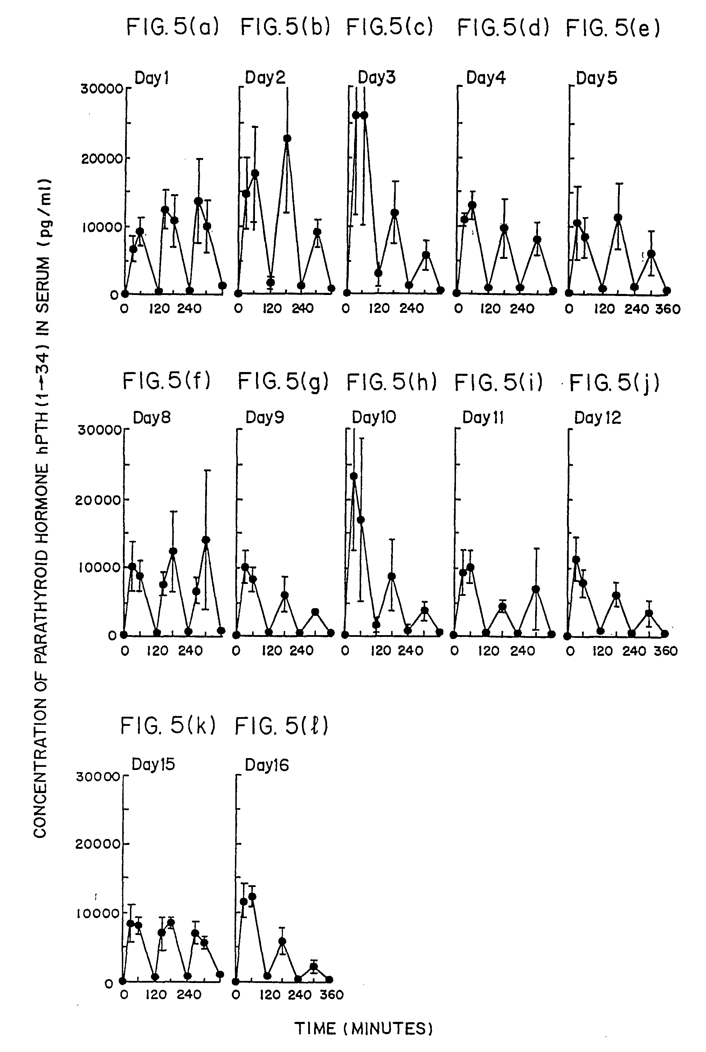

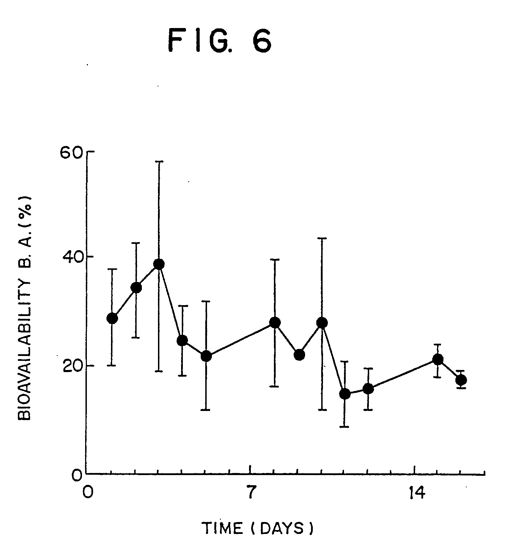

The results of Example 1 are shown in Figs. 5 and 6. Figs. 5(a) to 5(*l*) show a variation in the concentration of hPTH (1→34) in serum in relation to the time, revealing that a high peptide-in-serum concentration is ensured every administration in response to an electric current being applied and that the electric conductivity is maintained after the 16th day while suppressing the evaporation of moisture to keep a good response to the electric current being applied. In this way, it will be appreciated the obtained pulsatile serum hPTH (1→34) levels in response to the on/off currents are suitable for hPTH (1→34) to exert its action for bone formation and to cure the osteoporotic disease. Further, Fig. 6 shows a bioavailability (B. A.: %) which was calculated from an area under peptide-in-serum concentration-time curve (AUC) obtained by intravenous administration of hPTH (1→34) at the dose of 2 µg/kg. As shown in Fig. 6, a high bioavailability of hPTH (1→34) was maintained at every administration.

### Experimental Example 4

Using an electric conductor composition of Example 1 without addition of any L-proline (i.e. comprising 5 wt% of urea and 95 wt% of a citrate buffer solution (33 mM, pH = 5)) and the composition of Example 1, rats were iontophoretically administered under the same current application and administration conditions by use of the same type of applicator as in Experimental Example 1. The iontophoretic administration was performed on the same rats as used above one day and two days after the initial administration to observe whether or not local stimulation appeared at the administered site of each rat. Table 3 shows the results of the observation, in which 0 = not stimulated, 1 = slightly stimulated, 2 = moderately stimulated, and 3 = heavily stimulate. As shown in table 3, with the electric conductor composition free of any L-proline, a slight or moderate degree of local stimulation was recognized. On the other hand, the composition of Example 1 is fully free of any local stimulation.

**Table 3**

| | L-Proline Not Contained | | L-Proline Contained | | | |
|---|---|---|---|---|---|---|
| Rats | A | B | A | B | C | D |
| First Day | 0 | 2 | 0 | 0 | 0 | 0 |
| | | | | | | |
| Second Day | 1 | 2 | 0 | 0 | 0 | 0 |

### Experimental Example 5

In Experimental example 5, an applicator of Fig. 2 was used as in Experimental Example 2. Xanthan gum and locust bean gum as a thickener and benzoic acid and methylparahydroxy benzoate as a preservative were added to an electric conductor composition as shown below. For the iontophoretic administration test on rats, 200 µg of hPTH (1→34) was impregnated in the drug-retaining membrane 17 and a pulse direct current (50 kHz, 50% duty) was continuously applied over 60 minutes under a constant permeation current f 0.1 mA/cm²).

### Example 2

| Components | Content (%) |
|---|---|
| Urea | 5 |
| L-Proline | 10 |
| Agar | 1 |
| Xanthan gum | 0.25 |
| Locust bean gum | 0.25 |
| Cholestyramine | 5 |
| Benzoic acid | 0.2 |
| Methyl-p-hydroxy benzoate | 0.2 |
| Citric acid monohydrate | 0.25 |
| Trisodium citrate dihydrate | 0.7 |
| Distilled water for injection | 77.15 |
| Total | 100 |

### Example 3

| Components | Content (%) |
|---|---|
| Urea | 5 |
| L-Proline | 10 |
| Agar | 1 |
| Xanthan gum | 0.25 |
| Locust bean gum | 0.25 |
| Cholestyramine | 5 |
| Benzoic acid | 0.1 |
| Methyl-p-hydroxy benzoate | 0.2 |
| Trisodium citrate dihydrate | 0.2 |
| Distilled water for injection | 78.0 |
| Total | 100 |

### Example 4

| Components | Content (%) |
|---|---|
| Urea | 5 |
| L-Proline | 10 |
| Agar | 1 |
| Xanthan gum | 0.25 |
| Locust bean gum | 0.25 |
| Cholestyramine | 5 |
| Benzoic acid | 0.2 |
| Methyl-p-hydroxy benzoate | 0.2 |
| Trisodium citrate dihydrate | 0.25 |
| Distilled water for injection | 77.85 |
| Total | 100 |

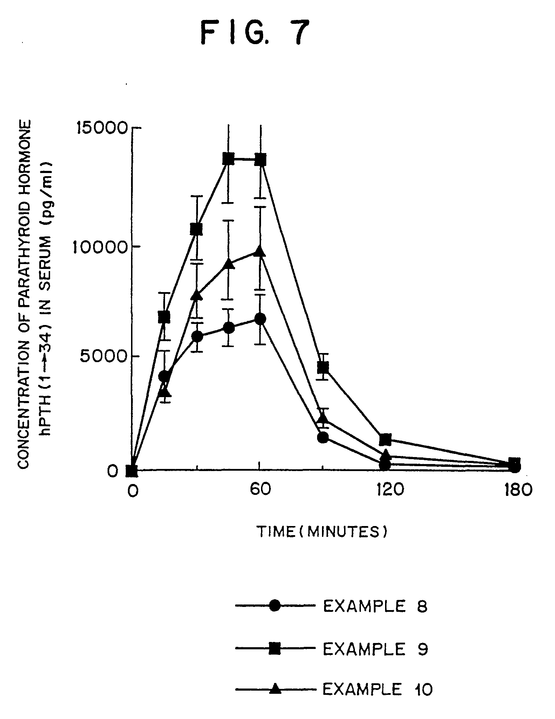

Fig. 7 shows a variation in the concentration of hPTH (1→34) in serum in relation to the time at the time when the electric conductor compositions of Examples 2 to 4 were each iontophoretically administered. All of the compositions exhibited good absorptivity in response to the electric current being applied. With respect to the compositions of Examples 2 and 4, bacteria and fungi were inoculated, followed by checking a variation in the number of bacteria and fungi to check preservative power of the formulated preservatives, with the result that the category II (i.e. the same level as or below the inoculated number of bacteria and fungi) which is based on the standards defined in the Japanese Pharmacopoeia XIII was satisfied.

As will be apparent from the foregoing, the drug administration composition for iontophoresis according to the present invention can appropriately suppress evaporation of moisture from a drug solution to keep electric conductivity thereof over a long time and promotes transdermal absorption of the drug. Accordingly, the drug can be safely, transdermally administered at a high absorption rate. Urea or N-methyl-2-pyrrolidone is preferred as a humectant. The combination of urea and N-methyl-2-pyrrolidone is more preferred. The use of L-proline as a co-humectant is more effective.

## Claims

1. A drug administration composition which comprises:
(i) An ionizable drug; and
(ii) a humectant selected from the group consisting of a mixture of at least one amide and L-proline, wherein the at least one amide is an amide other than a acidic amino acid amide.

2. A drug administration composition according to claim 1, wherein the humectant is present in an amount of 0.1 to 90% (w/w).

3. A drug administration composition according to claim 1 or 2, wherein the amide is selected from the group consisting of urea, dimethylacetamide, diethyltoluamide, dimethylformamide, dimethyloctamide, dimethyldecamide, biodecomposable urea (1-alkyl-4-imidazolin-2-on), N-methyl-2-pyrrolidone, 2-pyrrolidone, 1-lauryl-2-pyrrolidone, 1-methyl-4-carboxy-2-pyrrolidone, 1-hexyl-4-carboxy-2-pyrrolidone, 1-lauryl-4-carboxy-2-pyrrolidone, 1-hexyl-4-methoxycarbonyl-2-pyrrolidone, 1-hexyl-4-methoxycarbonyl-2-pyrrolidone, 1-lauryl-4-methoxycarbonyl-2-pyrrolidone N-cyclohexylpyrrolidone, N-dimethylaminopropylpyrrolidone, N-cocoalkylpyrrolidone, N-tallowalkylpyrrolidone, N-(2-hydroxyethyl)-2-pyrrolidone, 1-dodecylacacyclohepthan-2-on, 1-geranylazacycloheptan-2-on, 1-farnesylazacycloheptan-2-on, 1-(3,7-dimethyloctyl)azacycloheptan-2-on, 1-(3,7,11-dimethyldodecyl)azacycloheptan-2-on, 1-geranylazacyclohexan-2-on, 1-geranylazacycloheptan-2,5-dion, 1-farnesylazacyclopentan-2-on, 1-[2-(decylthio)ethyl]azacyclopentan-2-on.

4. A drug administration composition according to claim 1 or 2, wherein the amide is urea or a mixture of urea and N-methyl-2-pyrrolidone in an amount of 1-30% (w/w) of the composition.

5. A drug administration composition according to claim 1, wherein the humectant comprises L-proline in an amount of 1 to 30% (w/w) of the composition.

6. A drug administration composition according to claim 1, wherein the drug to be ionized is cationized in a buffer solution having a pH of 3 to 7.

7. A drug administration composition according to claim 6, wherein the buffer solution further comprises a hydrophilic aliphatic carboxylic acid having 2 to 6 carbon atoms, a salt and hydrate thereof.

8. A drug administration composition according to claim 1, wherein the drug is a biologically active peptide.

9. A drug administration composition according to claim 8, wherein the biologically active peptide consits of calcium-regulating hormone.

10. A drug administration composition according to claim 9, wherein the calcium-regulating hormone is a member selected from the group consisting of parathyroid hormone, its derivatives and salts thereof.

## Patentansprüche

1. Mittel zur Arzneistoffverabreichung, daß:
(i) einen ionisierbaren Arzneistoff; und
(ii) ein Befeuchtungsmittel ausgewählt aus der Gruppe, bestehend aus einem Gemisch von mindestens einem Amid und L-Prolin, wobei das mindestens eine Amid ein anderes Amid als ein saures Aminosäureamid ist, enthält

2. Mittel zur Arzneistoffverabreichung nach Anspruch 1, wobei das Befeuchtungsmittel in einer Menge von 0,1 bis 90% (w/w) enthalten ist.

3. Mittel zur Arzneistoffverabreichung nach Anspruch 1 oder 2, wobei das Amid ausgewählt ist aus der Gruppe, bestehend aus Harnstoff, Dimethylacetamid, Dimethyltoluamid, Dimethylformamid, Dimethyloctamid, Dimethyldecamid, biologisch abbaubarem Harnstoff (1-Alkyl-4-imidazolin-2-on), N-Methyl-2-pyrrolidon, 2-Pyrrolidon, 1-Lauryl-2-pyrrolidon, 1-Methyl-4-carboxy-2-pyrrolidon, 1-Hexyl-4-carboxy-2-pyrrolidon, 1-Lauryl-4-carboxy-2-pyrrolidon, 1-Hexyl-4-methoxycarbonyl-2-pyrrolidon, 1-Hexyl-4-methoxycarbonyl-2-pyrrolidon, 1-Lauryl-4-methoxycarbonyl-2-pyrrolidon N-Cyclohexylpyrrolidon, N-Dimethylaminopropylpyrrolidon, N-Cocoalkylpyrrolodin, N-Talgalkylpyrrolidon, N-(2-Hydroxyethyl)-2-pyrrolidon, 1-Dodecylacacycloheptan-2-on, 1-Geranylazacycloheptan-2-on, 1-Farnesylazacycloheptan-2-on, 1-(3,7-Dimethyloctyl)azacycloheptan-2-on, 1-(3,7,11-Dimethyldodecyl)azacycloheptan-2-on, 1-Geranylazacyclohexan-2-on, 1-Geranylazacycloheptan-2,5-dion, 1-Farnesylazacyclopentan-2-on, 1-[2-(decylthio)ethyl]azacyclopentan-2-on.

4. Mittel zur Arzneistoffverabreichung nach Anspruch 1 oder 2, wobei das Amid Harnstoff oder ein Gemisch aus Harnstoff und N-Methyl-2-pyrrolidon in einer Menge von 1-30% (w/w) des Mittels ist.

5. Mittel zur Arzneistoffverabreichung nach Anspruch 1, wobei das Befeuchtungsmittel L-Prolin in einer Menge von 1 bis 30% (w/w) des Mittels enthält.

6. Mittel zur Arzneistoffverabreichung nach Anspruch 1, wobei der Arzneistoff, der ionisiert werden soll, in einer Pufferlösung, die einen pH von 3 bis 7 hat, kationisiert wird.

7. Mittel zur Arzneistoffverabreichung nach Anspruch 6, wobei die Pufferlösung des Weiteren eine hydrophile, aliphatische Karbonsäure, die 2 bis 6 Kohlenstoffatome besitzt, ein Salz und ein Hydrat davon umfaßt.

8. Mittel zur Arzneistoffverabreichung nach Anspruch 1, wobei der Arzneistoff ein biologisch aktives Peptid ist.

9. Mittel zur Arzneistoffverabreichung nach Anspruch 8, wobei das biologisch aktive Peptid aus einem Kalzium-regulierenden Hormon besteht.

10. Mittel zur Arzneistoffverabreichung nach Anspruch 9, wobei das Kalziumregulierende Hormon ein Mitglied ausgewählt aus der Gruppe, bestehend aus Parathyroid-Hormon, seinen Derivaten und Salzen davon ist.

## Revendications

1. Composition pour l'administration d'un médicament qui comprend :
(i) Un médicament ionisable, et
(ii) un humectant choisi parmi le groupe constitué d'un mélange d'au moins un amide et la L-proline, dans lequel le au moins un amide est un amide autre qu'un amide d'acide aminé acide.

2. Composition pour l'administration d'un médicament selon la revendication 1, dans laquelle l'humectant est présent dans une proportion de 0,1 à 90 % (poids/poids).

3. Composition pour l'administration d'un médicament selon la revendication 1 ou 2, dans laquelle l'amide est choisi parmi le groupe constitué de l'urée, du diméthylacétamide, du diéthyltoluamide, du diméthylformamide, du diméthyloctamide, du diméthyldécamide, de l'urée biodécomposable (1-alkyl-4-imidazolin-2-one), de la N-méthyl-2-pyrrolidone, de la 2-pyrrolidone, de la 1-lauryl-2-pyrrolidone, de la 1-méthyl-4-carboxy-2-pyrrolidone, de la 1-hexyl-4-carboxy-2-pyrrolidone, de la 1-lauryl-4-carboxy-2-pyrrolidone, de la 1-hexyl-4-méthoxycarbonyl-2-pyrrolidone, de la 1-hexyl-4-méthoxycarbonyl-2-pyrrolidone, de la 1-lauryl-4-méthoxycarbonyl-2-pyrrolidone, de la N-cyclohexyl-pyrrolidone, de la N-diméthylaminopropylpyrrolidone, d'une N-cocoalkylpyrrolidone, d'une N-tallowalkylpyrrolidone, de la N-(2-hydroxyéthyl)-2-pyrrolidone, de la 1-dodécylacacycloheptan-2-one, de la 1-géranylazacycloheptan-2-one, de la 1-farnésylazacycloheptan-2-one, de la 1-(3,7-diméthyloctyl)azacycloheptan-2-one, de la 1-(3,7,11-diméthyldodécyl)azacycloheptan-2-one, de la 1-géranylazacyclohexan-2-one, de la 1-géranylazacycloheptan-2,5-dione, de la 1-farnésylazacyclopentan-2-one, de la 1-[2-(décylthio)éthyl]azacyclopentan-2-one.

4. Composition pour l'administration d'un médicament selon la revendication 1 ou 2, dans laquelle l'amide est de l'urée ou un mélange d'urée et de N-méthyl-2-pyrrolidone dans une proportion de 1 à 30 % (poids/poids) de la composition.

5. Composition pour l'administration d'un médicament selon la revendication 1, dans laquelle l'humectant comprend de la L-proline dans une proportion de 1 à 30 % (poids/poids) de la composition.

6. Composition pour l'administration d'un médicament selon la revendication 1, dans laquelle le médicament devant être ionisé est rendu cationique dans une solution tampon présentant un pH de 3 à 7.

7. Composition pour l'administration d'un médicament selon la revendication 6, dans laquelle la solution tampon comprend en outre un acide carboxylique aliphatique hydrophile comportant 2 à 6 atomes de carbone, un sel et un hydrate de celui-ci.

8. Composition pour l'administration d'un médicament selon la revendication 1, dans laquelle le médicament est un peptide biologiquement actif.

9. Composition pour l'administration d'un médicament selon la revendication 8, dans laquelle le peptide biologiquement actif est constitué d'une hormone de régulation du calcium.

10. Composition pour l'administration d'un médicament selon la revendication 9, dans laquelle l'hormone de régulation du calcium est un élément choisi parmi le groupe constitué de l'hormone parathyroïdienne, de ses dérivés et de sels de ceux-ci.
